# FASCICULE DE BREVET EUROPEEN

(11) **EP 2 395 959 B1**
(45) Date de publication et mention de la délivrance du brevet: **09.01.2013**
(21) Numéro de dépôt: 09774723.2
(22) Date de dépôt: 30.11.2009
(51) Int. Cl.: A61F 13/02

(54) **DISPOSITIF DE PROTECTION D'UNE ZONE DU CORPS HUMAIN NOTAMMENT POUR LA PREVENTION DES ESCARRES**
VORRICHTUNG ZUM SCHUTZ EINES BEREICHS DES MENSCHLICHEN KÖRPERS, INSBESONDERE ZUR VERHINDERUNG VON SCHORF
DEVICE FOR PROTECTING AN AREA OF THE HUMAN BODY, IN PARTICULAR FOR PREVENTING ESCHARS

(30) Priorité: 13.02.2009 FR 0900663
(43) Date de publication de la demande: 21.12.2011
(73) Titulaire: Millet Innovation, 26270 Loriol sur Drome (FR)
(72) Inventeur: DOMINGUEZ, Jean-Pierre, F-26300 Charpfey (FR); MILLET, Damien, F-26000 Valence (FR); MILLET, Jean-Claude, F-26800 Etoile sur Rhône (FR)
(74) Mandataire: de Roquemaurel, Bruno
(86) Numéro de dépôt international: PCT/IB2009/007628
(87) Numéro de publication internationale: WO 2010/092428

(56) Documents cités:
- EP-A- 0 300 620
- EP-A- 1 452 156
- WO-A-00/53139
- FR-A- 2 712 487
- FR-A- 2 904 932
- US-A- 4 655 767
- US-A1- 2005 113 732

## Description

La présente invention concerne la protection d'une zone de la peau notamment dans le but de prévenir la formation d'escarres. Plus généralement, la présente invention vise à apporter une protection mécanique et une hydratation d'une zone relativement grande de la peau, et notamment une zone de la peau fragilisée par exemple par des pathologies telles que le psoriasis ou l'eczéma, ou par une cicatrice récente.

Il est connu d'utiliser des plaquettes à base de gel polymère tel que du gel de silicone ou à base d'hydrogel pour assurer une fonction de protection de la peau ou de répartition de charge. Or les contraintes liées à l'effet recherché de la plaquette sur la peau s'avèrent souvent contradictoires avec les contraintes liées au maintien de la plaquette sur la zone à protéger ou à la manipulation, au transport et au stockage de la plaquette.

Par exemple pour assurer une fonction de répartition de charge, il est connu d'utiliser une plaquette en un gel de silicone de type PDMS (polydiméthylsiloxane) relativement dure. Des plaquettes en gel de silicone moins dures mais plus adhésives sont par exemple décrites dans les documents FR 2 712 487 et FR 2 904 932. Toutefois, les gels de silicone de ce type sont très peu adhésifs, et en tout état de cause, insuffisamment adhésifs pour tenir en place naturellement sur la zone à protéger. Par ailleurs, pour assurer une répartition de charge suffisante, la plaquette doit présenter une certaine épaisseur de plusieurs millimètres, ce qui s'oppose au maintien de la plaquette sur la zone à protéger. En effet, plus les bords de plaquette sont épais plus ils risquent d'être accrochés, entrainant l'arrachement de la plaquette.

Il est connu par exemple des documents US 4 655 767 et WO 00/53 139 de dissocier la fonction de traitement ou de protection de la fonction de maintien sur la peau, en utilisant une bande adhésive sur laquelle est disposé l'élément assurant la fonction de traitement ou de protection. Toutefois, cette solution ne s'avère pas appropriée notamment lorsque la surface à protéger est relativement grande, et lorsque la peau à protéger est fragile. En effet, les personnes soumises au risque d'escarres ont souvent la peau fragile, mal vascularisée et parfois sèche. Les adhésifs classiques sur film de polyuréthane risquent de provoquer une maltraitance de la peau (arrachements de morceaux de peau ou de poils) lors du retrait du film. Cette maltraitance est d'autant plus importante et pénalisante que la surface adhésive est grande. Il est également essentiel d'éviter tout risque d'allergie, ce qui est fréquent avec les solutions classiques.

Il est donc souhaitable de réaliser un dispositif de protection capable de protéger une relativement grande surface de peau, sans risque d'allergie, et qui soit susceptible de coller à la peau sans risque de décollement intempestif et sans risque de maltraitance de la peau lors du retrait du dispositif.

Des modes de réalisation peuvent concerner un dispositif de protection d'une zone de la peau du corps humain, comprenant une plaquette en un gel polymère comportant une face recouverte d'une couche externe, et une face opposée à la couche externe, destinée à être appliquée sur une zone de la peau à protéger. Selon un mode de réalisation, la plaquette comprend une partie protectrice en un gel polymère et au moins une partie latérale adhésive en un gel polymère, destinées à venir en contact avec la peau, les gels polymère formant la partie protectrice et la partie adhésive étant au moins partiellement mélangés dans une zone d'interface entre la partie protectrice et la partie adhésive, la partie protectrice étant configurée pour assurer une protection mécanique de la zone à protéger, le gel polymère formant la partie adhésive présentant un pouvoir adhésif supérieur à celui formant la partie protectrice pour assurer le maintien de la plaquette sur la zone à protéger.

Selon un mode de réalisation, la partie protectrice de la plaquette présente une épaisseur comprise entre 1 et 4 mm.

Selon un mode de réalisation, la partie adhésive présente une épaisseur comprise entre 0,2 et 0,5 mm.

Selon un mode de réalisation, la partie protectrice est réalisée par polymérisation au moins partielle d'un mélange comprenant : environ 15% de polydiméthylsiloxane à terminaisons diméthyl-vinyles ayant une viscosité supérieure à 20000 mPa.s, environ 25% de polydiméthylsiloxane à terminaisons diméthyl-vinyles ayant une viscosité comprise entre 200 et 20000 mPa.s, environ 45% de polydiméthylsiloxane à terminaisons triméthyles de ayant une viscosité inférieure à 100 mPa.s, environ 12% de silice de pyrogénation traitée triméthylsiloxy, et environ 3% de co-polydiméthylsiloxane-polyméthyl-hydrogéno-siloxane à terminaisons diméthyle-hydrogéno.

Selon un mode de réalisation, la partie protectrice est réalisée par polymérisation au moins partielle d'un mélange comprenant : environ 15% de polydiméthylsiloxane à terminaisons diméthyl-vinyles ayant une viscosité supérieure à 20000 mPa.s, environ 25% de polydiméthylsiloxane à terminaisons diméthyl-vinyles ayant une viscosité comprise entre 200 et 20000 mPa.s, environ 25% de polydiméthylsiloxane à terminaisons triméthyles de ayant une viscosité inférieure à 100 mPa.s, environ 20% de polydiméthylsiloxane à terminaisons triméthyles ayant une viscosité supérieure à 20000 mPa.s, environ 12% de silice de pyrogénation traitée triméthylsiloxy, et environ 3% de co-polydiméthylsiloxane-polyméthyl-hydrogéno-siloxane à terminaisons diméthyle-hydrogéno.

Selon un mode de réalisation, la partie adhésive est réalisée en Epithélium 28®.

Selon un mode de réalisation, la couche externe est en tissu et est collée sur une face de la partie protectrice et de la partie adhésive.

Selon un mode de réalisation, le tissu formant la couche externe est élastique.

Selon un mode de réalisation, lequel la couche externe est en un gel de silicone non adhésif d'épaisseur comprise entre 0,2 et 0,4 mm ou en une colle silicone d'épaisseur comprise entre 0,05 et 0,4 mm.

Selon un mode de réalisation, chaque partie adhésive présente une largeur comprise entre 5 et 60 mm et un pouvoir adhésif de l'ordre de 130 à 140 g/cm2.

Des modes de réalisation peuvent concerner également un procédé de fabrication d'un dispositif de protection d'une zone de la peau du corps humain. Selon un mode de réalisation, le procédé comprend des étapes de formation d'une plaquette en un gel polymère, et de fixation d'une couche externe sur une face de la plaquette, la plaquette comprenant une partie protectrice en un gel polymère et au moins une partie latérale adhésive en un gel polymère, destinées à venir en contact avec la peau, les gels polymère formant la partie protectrice et la partie adhésive étant au moins partiellement mélangés dans une zone d'interface entre la partie protectrice et la partie adhésive, la partie protectrice étant configurée pour assurer une protection mécanique de la zone à protéger, le gel polymère formant la partie adhésive présentant un pouvoir adhésif supérieur à celui formant la partie protectrice, pour assurer le maintien de la plaquette sur la zone à protéger.

Selon un mode de réalisation, le procédé comprend des étapes de formation de la partie adhésive à partir d'un premier mélange liquide déposé sur un support, et de formation de la partie protectrice à partir d'un second mélange liquide déposé sur le support, le premier mélange et le second mélange se mélangeant partiellement dans une zone d'interface, avant polymérisation complète des deux mélanges.

Selon un mode de réalisation, le second mélange comprend : environ 15% de polydiméthylsiloxane à terminaisons diméthyl-vinyles ayant une viscosité supérieure à 20000 mPa.s, environ 25% de polydiméthylsiloxane à terminaisons diméthyl-vinyles ayant une viscosité comprise entre 200 et 20000 mPa.s, environ 45% de polydiméthylsiloxane à terminaisons triméthyles de ayant une viscosité inférieure à 100 mPa.s, environ 12% de silice de pyrogénation traitée triméthylsiloxy, et environ 3% de co-polydiméthylsiloxane-polyméthyl-hydrogéno-siloxane à terminaisons diméthyle-hydrogéno.

Selon un mode de réalisation, le second mélange comprend : environ 15% de polydiméthylsiloxane à terminaisons diméthyl-vinyles ayant une viscosité supérieure à 20000 mPa.s, environ 25% de polydiméthylsiloxane à terminaisons diméthyl-vinyles ayant une viscosité comprise entre 200 et 20000 mPa.s, environ 25% de polydiméthylsiloxane à terminaisons triméthyles de ayant une viscosité inférieure à 100 mPa.s, environ 20% de polydiméthylsiloxane à terminaisons triméthyles ayant une viscosité supérieure à 20000 mPa.s, environ 12% de silice de pyrogénation traitée triméthylsiloxy, et environ 3% de co-polydiméthylsiloxane-polyméthyl-hydrogéno-siloxane à terminaisons diméthyle-hydrogéno.

Selon un mode de réalisation, les deux mélanges liquides sont déposés sur un support défilant de manière à former des bandes, le premier mélange étant déposé sur le support de manière à former deux bandes adhésives entre lesquelles le second mélange est déposé pour former la partie protectrice sous forme de bande.

Selon un mode de réalisation, l'épaisseur et la largeur de chacune des parties protectrice et adhésive sont ajustées par une raclette et des guides latéraux.

Selon un mode de réalisation, le procédé comprend une étape de fixation sur une face de la plaquette opposée à la face destinée à venir en contact avec la peau d'une couche externe non adhésive.

Selon un mode de réalisation, le procédé comprend une étape de collage de la couche externe en tissu sur la plaquette.

Selon un mode de réalisation, la couche externe est formée en un gel polymère non adhésif d'épaisseur comprise entre 0,2 et 0,4 mm ou en une colle silicone d'épaisseur comprise entre 0,05 et 0,4 mm.

Selon un mode de réalisation, le procédé comprend une étape de couverture de la face de la plaquette destinée à venir en contact avec la peau d'un film protecteur.

Des exemples de réalisation de l'invention seront décrits dans ce qui suit, à titre non limitatif en relation avec les figures jointes parmi lesquelles :
- la figure 1 représente schématiquement un dispositif de protection selon un mode de réalisation,
- la figure 2 représente schématiquement en vue de profil une machine de fabrication du dispositif de protection, selon un mode de réalisation,

- la figure 3 est une vue schématique de dessus de la machine de fabrication représentée sur la figure 2,
- la figure 4 représente schématiquement un dispositif de protection selon un autre mode de réalisation,
- la figure 5 représente schématiquement le dispositif de protection de la figure 4 dans une configuration prête à l'emploi.

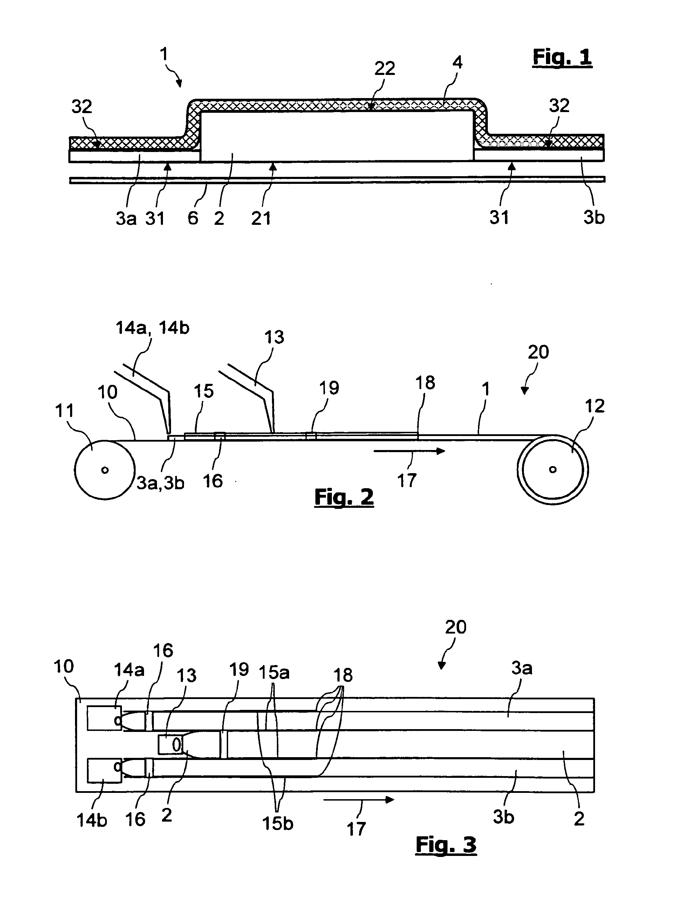

La figure 1 représente un dispositif de protection selon un mode de réalisation. Sur la figure 1, le dispositif de protection se présente sous la forme d'une plaquette 1 comprenant une partie centrale 2 prévue pour couvrir une zone à protéger de la peau du corps humain, et des parties latérales 3a, 3b comportant une face arrière 32 et une face avant adhésive 31 destinée à venir en contact avec la peau et à maintenir la plaquette 1 sur la zone à protéger. La partie centrale 2 présente une face avant 21 prévue pour venir en contact avec la peau, une face arrière 22 et une épaisseur adaptée aux propriétés de protection mécanique recherchées. L'épaisseur des parties latérales 3a, 3b peut être plus petite que celle de la partie centrale 2. Les parties latérales sont solidaires de la partie centrale de manière à ce que la face avant 21 de la partie centrale soit située sensiblement dans le même plan que la face avant 31 des parties latérales. Les faces arrières 22 et 32 de la partie centrale et des parties latérales sont recouvertes d'une couche de protection 4.

Les parties latérales 3a, 3b peuvent être réalisées en un gel de silicone réticulé pour être suffisamment adhésif, par exemple en Epithélium 28® commercialisé par la société Millet Innovation. Ce matériau se caractérise par une très grande adhésivité, sans risque de provoquer des arrachements de peau ou de poils lors du retrait du dispositif de protection, et ne risque pas de provoquer d'allergies. L'épaisseur les parties latérales 3a, 3b peut être relativement faible, par exemple comprise entre 0,2 et 0,5 mm.

La partie centrale 2 peut être réalisée en un gel polymère, par exemple un gel de silicone. Si la partie centrale 2 doit assurer une protection mécanique, elle est réalisée en un gel de silicone réticulé pour être relativement dur et donc faiblement adhésif. La partie centrale 2 présente une épaisseur qui peut être une dizaine de fois plus grande que celle des parties latérales 3a, 3b.

Dans une application à la prévention contre les escarres, la partie centrale 2 est réalisée en un gel de silicone, présentant une épaisseur comprise entre 1 à 4 mm d'épaisseur, et des propriétés de rigidité, de viscosité et de facteur d'amortissement aptes à limiter les cisaillements sous-jacents et maintenir une microcirculation dans les tissus protégés, malgré la présence d'une pression. A cet effet, le gel de silicone peut présenter à 35°C une rigidité ou composante élastique variant d'environ 11000 à 20000 Pa, une viscosité ou composante amortissante variant d'environ 700 à 8000 Pa et un facteur d'amortissement variant d'environ 0,06 à 0,38, lorsqu'un taux de cisaillement varie entre 0 et 100 rd/s.

Ces propriétés sont par exemple obtenues lorsque la partie centrale 2 est réalisée en polymérisant au moins partiellement l'un ou l'autre de deux mélanges dont la composition est résumée dans le tableau 1 suivant :

**Tableau 1**

| Composant | Mélange 1 | Mélange 2 |
|---|---|---|
| polydiméthylsiloxane à terminaisons diméthyl-vinyles de viscosité supérieure à 20000 mPa.s | environ 15% | environ 15% |
| polydiméthylsiloxane à terminaisons diméthyl-vinyles de viscosité entre 200 et 20000 mPa.s | environ 25% | environ 25% |
| Polydiméthylsiloxane à terminaisons triméthyles de viscosité inférieure à 100 mPa.s | environ 45% | environ 25% |
| polydiméthylsiloxane à terminaisons triméthyles de haute viscosité supérieure à 20000 mPa.s | 0 | environ 20% |
| silice de pyrogénation traitée triméthylsiloxy | environ 12% | environ 12% |
| co-polydiméthylsiloxane-polyméthyl-hydrogéno-siloxane à terminaisons diméthyle-hydrogéno | environ 3% | environ 3% |

La polymérisation partielle des mélanges 1 et 2 peut être obtenue à l'aide d'un système catalytique à base de platine complexé vinylesiloxanes. Lorsqu'elle est réalisée à partir des mélanges 1 et 2, la partie centrale 2 présente une adhésivité comprise entre 100 à 115 g/cm2.

La couche de protection 4 peut être réalisée en un tissu, par exemple un tissu élastique, tel qu'un tissu en polyamide élasthanne.

Un film protecteur 6 peut être disposé sur les faces 21 et 31, destinées à venir en contact avec la peau, pour permettre le stockage et le transport du dispositif de protection 1, le film protecteur étant retiré juste avant l'application du dispositif 1 sur la peau.

Les figures 2 et 3 représentent une machine de fabrication 20 du dispositif de protection. La machine 20 permet de fabriquer des plaquettes 1 sous la forme d'une bande continue. La machine 20 comprend une bande support 10 anti-adhésive, par exemple en PVC, qui est déroulée d'un côté d'une bobine 11 et enroulée de l'autre autour d'une autre bobine 12. Entre les deux bobines 11, 12, la bande 10 se déplace sensiblement horizontalement dans un sens de défilement indiqué par la flèche 17. La machine 20 comprend au moins deux buses latérales 14a, 14b et une ou plusieurs buses centrales 13 qui peuvent être installées à une certaine distance des buses latérales vers la bobine 12. Les buses 14a, 14b permettent de former les parties latérales 3a, 3b de la plaquette 1. A cet effet, les buses 14a, 14b déposent sur la bande 10 un premier mélange d'huiles de silicone entre des guides latéraux, internes 15a et externes 15b disposés longitudinalement et verticalement sur la bande 10. La buse 13 permet de former la partie centrale 2 de la plaquette 1. A cet effet, la buse 13 dépose entre les deux guides internes 15a un second mélange d'huiles de silicone. Le débit des buses 13, 14a, 14b est adapté au temps de réticulation des mélanges, à la vitesse de défilement de la bande 10 et aux dimensions des parties centrale 2 et latérales 3a, 3b.

La machine de fabrication 20 comprend également des raclettes 16, 19 disposées horizontalement au-dessus de la bande 10 pour ajuster l'épaisseur des parties latérales 3a, 3b et de la partie centrale 2. La combinaison des guides 15a, 15b et des raclettes permet d'ajuster finement les largeurs et épaisseurs respectives des parties centrale 2 et latérales 3a, 3b.

Le premier mélange est conçu pour obtenir des conditions d'étalement particulières sur la bande 10, un certain temps de polymérisation et des propriétés viscoélastiques et d'adhésivité finales appropriées. Le second mélange est conçu pour obtenir des propriétés viscoélastiques correspondant à la fonction à obtenir du dispositif de protection à réaliser. Les premier et second mélanges contiennent par exemple du PDMS (polydiméthylsiloxane).

A l'extrémité aval 18 (par rapport au sens de défilement 17) des guides 15a, 15b, les parties latérales 3a, 3b dont la viscosité est devenue plus importante que celle du second mélange retiennent en partie la partie centrale 2 moins visqueuse. Comme la partie centrale 2 est plus épaisse que les partis latérales 3a, 3b, le second mélange a tendance à recouvrir dans une zone d'interface de faible largeur les parties latérales 3a, 3b. Dans cette zone d'interface, les huiles de silicone non encore polymérisées des deux mélanges ont tendance à se mélanger. On obtient donc après polymérisation une continuité complète entre la partie centrale et les parties latérales. A noter également que le premier mélange formant les parties latérales 3a, 3b polymérise plus vite que le second mélange formant la partie centrale 2.

En aval des guides 15a, 15b, la polymérisation des deux mélanges se poursuit par exemple dans un tunnel de polymérisation (non représenté) qui peut être chauffé pour accélérer la polymérisation et donc la cadence de production du dispositif de protection. A la fin de la polymérisation, l'ensemble de la plaquette 1 en bande et de la bande support 10 est enroulé sur la bobine 12. La bobine 12 est ensuite placée dans une machine de collage en continu de la couche externe 4 sur les faces 22 et 32 des parties centrale et latérales, et d'application sur les faces 21 et 31 opposées du film protecteur 6.

La plaquette 1 présente un pouvoir adhésif suffisant lorsque les parties latérales 3a, 3b présentent une largeur comprise entre 5 et 60 mm et un pouvoir adhésif de l'ordre de 130 à 140 g/cm2.

Par ailleurs, il est à noter que le premier mélange peut être alternativement déposé sur la bande support 10 après le second mélange.

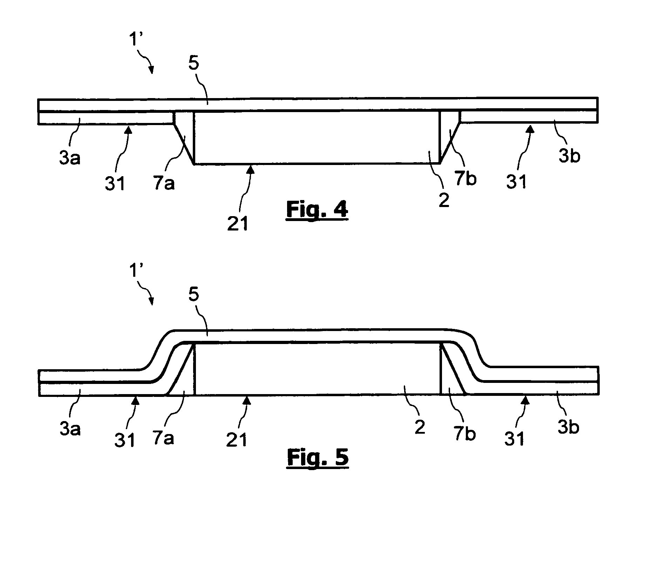

La figure 4 représente une plaquette de protection 1' selon un autre mode de réalisation. La plaquette 1' diffère de la plaquette 1 représentée sur la figure 1 en ce que la couche de tissu 4 est remplacée par une couche en gel polymère 5 relativement dure et non adhésive. La couche 5 peut être formée dans la machine de fabrication avant les parties latérales 3a, 3b et centrale 2, les premier et second mélanges étant coulés sur la couche 5 après polymérisation au moins partielle de cette dernière, de manière à obtenir un mélange des couches dans des zones d'interface et ainsi une continuité complète entre la couche 5 et les parties centrale 2 et latérales 3a, 3b. Les parties latérales 3a, 3b se trouvent ainsi liées à la partie centrale 2 par des zones de jonction 7a, 7b constituées d'un matériau différent. La couche 5 peut être réalisée en un gel de silicone, par exemple du type Epithélium 26® commercialisé par la société Millet Innovation, et présente une épaisseur comprise entre 0,2 et 0,5 mm. La couche de protection 5 peut aussi être réalisée en une fine épaisseur de colle silicone telle qu'une colle commercialisée pour réaliser des assemblages de silicones, dont l'adhésivité ne joue pas ou très peu sur la bande support 10. La couche 5 peut ainsi présenter une épaisseur encore plus faible et être quasiment transparente.

La figure 5 représente la plaquette 1' dans une configuration prête à l'emploi, ou lors de l'application du film protecteur 6 sur les faces 21 et 31 des parties centrale 2 et latérales 3a, 3b. Dans la configuration représentée sur la figure 4, les faces 21 de la partie centrale 2 et 31 des parties latérales ne sont pas dans le même plan en raison de la méthode de fabrication mise en oeuvre. Dans la configuration représentée sur la figure 5, les parties latérales 3a, 3b et la couche 5 ont été déformées de manière à ce que les faces 31 soient au moins en partie dans le plan de la face 21.

La bande obtenue par la machine 10 est utilisée en découpant tout d'abord un morceau de la bande à une longueur correspondant à celle de la zone de peau à protéger, pour obtenir une plaquette individuelle 1 ou 1'. Le film de protection 6 est ensuite retiré de la plaquette qui est ensuite appliquée sur la zone à protéger. L'adhésivité et la finesse des parties latérales 3a, 3b de la plaquette 1 ou 1' permet un excellent maintien de la plaquette sur la peau. Par ailleurs, lorsque la partie centrale 2 est réalisée en un gel de silicone suffisamment épais et dur pour assurer une bonne répartition de charge, elle s'avère capable d'absorber une partie substantielle des efforts de cisaillement et de compression que peuvent subir les tissus au voisinage d'un os. La partie centrale 2 s'avère également capable de réduire les sollicitations dynamiques répétées des tissus qui entrainent une fatigue de la peau et en profondeur, des tissus et des vaisseaux. Elle peut donc également maintenir l'irrigation des tissus. L'adhésivité de la partie centrale, bien que faible, s'avère également suffisante pour limiter d'une manière importante les frottements entre la peau et son environnement. La partie centrale 2 s'avère donc efficace pour prévenir la formation d'escarres. Ce caractère protecteur est renforcé par le fait que le gel de silicone est relativement étanche à l'eau et favorise donc l'hydratation de la peau en contact par exsudation. Il en résulte une amélioration de la vascularisation superficielle de la peau en contact, ce qui tend à prévenir la formation d'escarres. A noter que la plaquette 1 ou 1' peut être facilement détachée de la peau, sans risque de formation de lésions même avec une peau fragile. La plaquette 1 ou 1' peut également être nettoyée facilement pour être réutilisée, le nettoyage n'affectant pas le caractère adhésif des parties latérales 3a, 3b.

Le dispositif de protection précédemment décrit peut être également utilisé dans d'autres buts, par exemple pour résorber des cicatrices chéloïdes ou protéger des zones de la peau atteintes de psoriasis ou d'eczémas.

Il apparaîtra clairement à l'homme de l'art que la présente invention est susceptible de diverses variantes de réalisation. En particulier, l'invention n'est pas limitée à un dispositif de protection fabriqué en bande à découper. Le dispositif de protection peut être fabriqué directement sous la forme de plaquettes de différentes dimensions, en versant des quantités adéquates des mélanges liquides dans des moules individuels avec des guides mobiles pour séparer les mélanges au début de leur polymérisation.

## Revendications

1. Dispositif de protection d'une zone de la peau du corps humain, comprenant une plaquette (1, 1') en un gel polymère comportant une face (22, 32) recouverte d'une couche externe (4, 5), et une face (21, 31) opposée à la couche externe, destinée à être appliquée sur une zone de la peau à protéger,
**caractérisé en ce que** la plaquette (1, 1') comprend une partie protectrice (2) en un gel polymère et au moins une partie latérale adhésive en un gel polymère (3a, 3b), destinées à venir en contact avec la peau, les gels polymère formant la partie protectrice et la partie adhésive étant au moins partiellement mélangés dans une zone d'interface entre la partie protectrice et la partie adhésive, la partie protectrice étant configurée pour assurer une protection mécanique de la zone à protéger, le gel polymère formant la partie adhésive présentant un pouvoir adhésif supérieur à celui formant la partie protectrice pour assurer le maintien de la plaquette sur la zone à protéger.

2. Dispositif selon la revendication 1, dans lequel la partie protectrice (2) de la plaquette (1, 1') présente une épaisseur comprise entre 1 et 4 mm.

3. Dispositif selon la revendication 1 ou 2, dans lequel la partie adhésive (3a, 3b) présente une épaisseur comprise entre 0,2 et 0,5 mm.

4. Dispositif selon l'une des revendications 1 à 3, dans lequel la partie protectrice (2) est réalisée par polymérisation au moins partielle d'un mélange comprenant :
environ 15% de polydiméthylsiloxane à terminaisons diméthyl-vinyles ayant une viscosité supérieure à 20000 mPa.s,
environ 25% de polydiméthylsiloxane à terminaisons diméthyl-vinyles ayant une viscosité comprise entre 200 et 20000 mPa.s,
environ 45% de polydiméthylsiloxane à terminaisons triméthyles de ayant une viscosité inférieure à 100 mPa.s,
environ 12% de silice de pyrogénation traitée triméthylsiloxy, et
environ 3% de co-polydiméthylsiloxane-polyméthyl-hydrogéno-siloxane à terminaisons diméthyle-hydrogéno.

5. Dispositif selon l'une des revendications 1 à 3, dans lequel la partie protectrice (2) est réalisée par polymérisation au moins partielle d'un mélange comprenant :
environ 15% de polydiméthylsiloxane à terminaisons diméthyl-vinyles ayant une viscosité supérieure à 20000 mPa.s,
environ 25% de polydiméthylsiloxane à terminaisons diméthyl-vinyles ayant une viscosité comprise entre 200 et 20000 mPa.s,
environ 25% de polydiméthylsiloxane à terminaisons triméthyles de ayant une viscosité inférieure à 100 mPa.s,
environ 20% de polydiméthylsiloxane à terminaisons triméthyles ayant une viscosité supérieure à 20000 mPa.s,
environ 12% de silice de pyrogénation traitée triméthylsiloxy, et
environ 3% de co-polydiméthylsiloxane-polyméthyl-hydrogéno-siloxane à terminaisons diméthyle-hydrogéno.

6. Dispositif selon l'une des revendications 1 à 5, dans lequel la couche externe (4) est en tissu et est collée sur une face de la partie protectrice (2) et de la partie adhésive (3a, 3b).

7. Dispositif selon la revendication 6, dans lequel le tissu formant la couche externe (4) est élastique.

8. Dispositif selon l'une des revendications 1 à 5, dans lequel la couche externe (5) est en un gel de silicone non adhésif d'épaisseur comprise entre 0,2 et 0,4 mm ou en une colle silicone d'épaisseur comprise entre 0,05 et 0,4 mm.

9. Dispositif selon l'une des revendications 1 à 7, dans lequel chaque partie adhésive (3a, 3b) présente une largeur comprise entre 5 et 60 mm et un pouvoir adhésif de l'ordre de 130 à 140 g/cm2.

10. Procédé de fabrication d'un dispositif de protection d'une zone de la peau du corps humain, **caractérisé en ce qu'**il comprend des étapes de formation d'une plaquette (1, 1') en un gel polymère, et de fixation d'une couche externe (4, 5) sur une face (22, 32) de la plaquette, la plaquette comprenant une partie protectrice (2) en un gel polymère et au moins une partie latérale adhésive (3a, 3b) en un gel polymère, destinées à venir en contact avec la peau, les gels polymère formant la partie protectrice et la partie adhésive étant au moins partiellement mélangés dans une zone d'interface entre la partie protectrice et la partie adhésive, la partie protectrice étant configurée pour assurer une protection mécanique de la zone à protéger, le gel polymère formant la partie adhésive présentant un pouvoir adhésif supérieur à celui formant la partie protectrice, pour assurer le maintien de la plaquette sur la zone à protéger.

11. Procédé selon la revendication 10, comprenant des étapes de formation de la partie adhésive (3a, 3b) à partir d'un premier mélange liquide déposé sur un support, (10), et de formation de la partie protectrice (2) à partir d'un second mélange liquide déposé sur le support, le premier mélange et le second mélange se mélangeant partiellement dans une zone d'interface (7a,7b), avant polymérisation complète des deux mélanges.

12. Procédé selon la revendication 11, dans lequel le second mélange comprend:
environ 15% de polydiméthylsiloxane à terminaisons diméthyl-vinyles ayant une viscosité supérieure à 20000 mPa.s,
environ 25% de polydiméthylsiloxane à terminaisons diméthyl-vinyles ayant une viscosité comprise entre 200 et 20000 mPa.s,
environ 45% de polydiméthylsiloxane à terminaisons triméthyles de ayant une viscosité inférieure à 100 mPa.s,
environ 12% de silice de pyrogénation traitée triméthylsiloxy, et
environ 3% de co-polydiméthylsiloxane-polyméthyl-hydrogéno-siloxane à terminaisons diméthyle-hydrogéno.

13. Procédé selon la revendication 11, dans lequel le second mélange comprend:
environ 15% de polydiméthylsiloxane à terminaisons diméthyl-vinyles ayant une viscosité supérieure à 20000 mPa.s,
environ 25% de polydiméthylsiloxane à terminaisons diméthyl-vinyles ayant une viscosité comprise entre 200 et 20000 mPa.s,
environ 25% de polydiméthylsiloxane à terminaisons triméthyles de ayant une viscosité inférieure à 100 mPa.s,
environ 20% de polydiméthylsiloxane à terminaisons triméthyles ayant une viscosité supérieure à 20000 mPa.s,
environ 12% de silice de pyrogénation traitée triméthylsiloxy, et
environ 3% de co-polydiméthylsiloxane-polyméthyl-hydrogéno-siloxane à terminaisons diméthyle-hydrogéno.

14. Procédé selon la revendication 13, dans lequel les deux mélanges liquides sont déposés sur un support défilant (10) de manière à former des bandes, le premier mélange étant déposé sur le support de manière à former deux bandes adhésives (3a, 3b) entre lesquelles le second mélange est déposé pour former la partie protectrice (2) sous forme de bande.

15. Procédé selon la revendication 14, dans lequel l'épaisseur et la largeur de chacune des parties protectrice (2) et adhésive (3a, 3b) sont ajustées par une raclette (16, 19) et des guides latéraux (15a, 15b).

16. Procédé selon l'une des revendications 10 à 15, comprenant une étape de fixation sur une face de la plaquette (1, 1') opposée à la face (21, 31) destinée à venir en contact avec la peau d'une couche externe (4, 5) non adhésive.

17. Procédé selon la revendication 16, comprenant une étape de collage de la couche externe (4) en tissu sur la plaquette (1).

18. Procédé selon la revendication 16, dans lequel la couche externe (5) est formée en un gel polymère non adhésif d'épaisseur comprise entre 0,2 et 0,4 mm ou en une colle silicone d'épaisseur comprise entre 0,05 et 0,4 mm.

19. Procédé selon l'une des revendications 10 a 18, comprenant une étape de couverture de la face (21, 31) de la plaquette (1, 1') destinée à venir en contact avec la peau d'un film protecteur (6).

## Claims

1. A device for protecting an area of the skin of the human body, comprising a pad (1, 1') of a polymer gel comprising a face (22, 32) covered by an external layer (4, 5), and a face (21, 31) opposite the external layer, intended to be applied onto an area of the skin to be protected,
**characterized in that** the pad (1, 1') comprises a protective part (2) of a polymer gel and at least one lateral adhesive part (3a, 3b) of a polymer gel, intended to come into contact with the skin, the polymer gels forming the protective part and the adhesive part being at least partially mixed in an interface area between the protective part and the adhesive part, the protective part being configured to ensure mechanical protection of the area to be protected, the polymer gel forming the adhesive part having an adhesive power greater than that forming the protective part to hold the pad on the area to be protected.

2. Device according to claim 1, wherein the protective part (2) of the pad (1, 1') has a thickness between 1 and 4 mm.

3. Device according to claim 1 or 2, wherein the adhesive part (3a, 3b) has a thickness between 0.2 and 0.5 mm.

4. Device according to one of claims 1 to 3, wherein the protective part (2) is produced by at least partially polymerizing a mixture comprising:
approximately 15% of dimethyl-vinyl-terminated polydimethylsiloxane having a viscosity greater than 20,000 mPa.s,
approximately 25% of dimethyl-vinyl-terminated polydimethylsiloxane having a viscosity between 200 and 20,000 mPa.s,
approximately 45% of trimethyl-terminated polydimethylsiloxane having a viscosity lower than 100 mPa.s,
approximately 12% of trimethylsiloxy-treated pyrogenic silica, and
approximately 3% of dimethyl-hydrogen-terminated co-polydimethylsiloxane-polymethyl-hydrogen-siloxane.

5. Device according to one of claims 1 to 3, wherein the protective part (2) is produced by at least partially polymerizing a mixture comprising:
approximately 15% of dimethyl-vinyl-terminated polydimethylsiloxane having a viscosity greater than 20,000 mPa.s,
approximately 25% of dimethyl-vinyl-terminated polydimethylsiloxane having a viscosity between 200 and 20,000 mPa.s,
approximately 25% of trimethyl-terminated polydimethylsiloxane having a viscosity lower than 100 mPa.s,
approximately 20% of trimethyl-terminated polydimethylsiloxane having a viscosity greater than 20,000 mPa.s,
approximately 12% of trimethylsiloxy-treated pyrogenic silica, and
approximately 3% of dimethyl-hydrogen terminated co-polydimethylsiloxane-polymethyl-hydrogen-siloxane.

6. Device according to one of claims 1 to 5, wherein the external layer (4) is made of fabric and is glued onto one face of the protective part (2) and of the adhesive part (3a, 3b).

7. Device according to claim 6, wherein the fabric forming the external layer (4) is elastic.

8. Device according to one of claims 1 to 5, wherein the external layer (5) is made of a non-adhesive silicone gel with a thickness between 0.2 and 0.4 mm or of a silicone glue with a thickness between 0.05 and 0.4 mm.

9. Device according to one of claims 1 to 7, wherein each adhesive part (3a, 3b) has a width between 5 and 60 mm and an adhesive power in the order of 130 to 140 g/cm2.

10. A method for manufacturing a device for protecting an area of the skin of the human body, **characterized in that** it comprises steps of forming a pad (1, 1') of a polymer gel, and of fixing an external layer (4, 5) on a face (22, 32) of the pad, the pad comprising a protective part (2) of a polymer gel and at least one lateral adhesive part (3a, 3b) of a polymer gel, intended to come into contact with the skin, the polymer gels forming the protective part and the adhesive part being at least partially mixed in an interface area between the protective part and the adhesive part, the protective part being configured to ensure mechanical protection of the area to be protected, the polymer gel forming the adhesive part having an adhesive power greater than that forming the protective part, to hold the pad on the area to be protected.

11. Method according to claim 10, comprising steps of forming the adhesive part (3a, 3b) from a first liquid mixture deposited on a support (10), and of forming the protective part (2) from a second liquid mixture deposited on the support, the first mixture and the second mixture partially mixing in an interface area (7a,7b), before full polymerization of both mixtures.

12. Method according to claim 11, wherein the second mixture comprises:
approximately 15% of dimethyl-vinyl-terminated polydimethylsiloxane having a viscosity greater than 20,000 mPa.s,
approximately 25% of dimethyl-vinyl-terminated polydimethylsiloxane having a viscosity between 200 and 20,000 mPa.s,
approximately 45% of trimethyl-terminated polydimethylsiloxane having a viscosity lower than 100 mPa.s,
approximately 12% of trimethylsiloxy-treated pyrogenic silica, and
approximately 3% of dimethyl-hydrogen terminated co-polydimethylsiloxane-polymethyl-hydrogen-siloxane.

13. Method according to claim 11, wherein the second mixture comprises:
approximately 15% of dimethyl-vinyl-terminated polydimethylsiloxane having a viscosity greater than 20,000 mPa.s,
approximately 25% of dimethyl-vinyl-terminated polydimethylsiloxane having a viscosity between 200 and 20,000 mPa.s,
approximately 25% of trimethyl-terminated polydimethylsiloxane having a viscosity lower than 100 mPa.s,
approximately 20% of trimethyl-terminated polydimethylsiloxane having a viscosity greater than 20,000 mPa.s,
approximately 12% of trimethylsiloxy-treated pyrogenic silica, and
approximately 3% of dimethyl-hydrogen-terminated co-polydimethylsiloxane-polymethyl-hydrogen-siloxane.

14. Method according to claim 13, wherein the two liquid mixtures are deposited on a moving or translating support (10) so as to form strips, the first mixture being deposited on the support so as to form two adhesive strips (3a, 3b) between which the second mixture is deposited to form the protective part (2) in strip form.

15. Method according to claim 14, wherein the thickness and the width of each of the protective (2) and adhesive (3a, 3b) parts are adjusted by a scraper (16, 19) and lateral guides (15a, 15b).

16. Method according to one of claims 10 to 15, comprising a step of fixing a non-adhesive external layer (4, 5) onto a face of the pad (1, 1') opposite the face (21, 31) intended to come into contact with the skin.

17. Method according to claim 16, comprising a step of gluing the external layer (4) of a fabric onto the pad (1).

18. Method according to claim 16, wherein the external layer (5) is formed of a non-adhesive polymer gel with a thickness between 0.2 and 0.4 mm or of a silicone glue with a thickness between 0.05 and 0.4 mm.

19. Method according to one of claims 10 to 18, comprising a step of covering the face (21, 31) of the pad (1, 1') intended to come into contact with the skin by a protective film (6).

## Patentansprüche

1. Vorrichtung zum Schutz eines Bereichs der Haut des menschlichen Körpers, umfassend ein Plättchen (1, 1') aus einem Polymer-Gel, das eine mit einer Außenschicht (4, 5) bedeckte Seite (22, 32) und eine der Außenschicht gegenüberliegende Seite (21, 31) umfasst, die zum Anbringen auf einem Bereich der zu schützenden Haut bestimmt ist,
**dadurch gekennzeichnet, dass** das Plättchen (1, 1') ein Schutzteil (2) aus einem Polymer-Gel und wenigstens ein klebendes Seitenteil aus einem Polymer-Gel (3a, 3b) umfasst, die bestimmt sind, mit der Haut in Kontakt zu kommen, wobei die das Schutzteil und das Klebeteil bildenden Polymer-Gele wenigstens teilweise in einem Schnittstellenbereich zwischen dem Schutzteil und dem Klebeteil vermischt sind, wobei das Schutzteil so konfiguriert ist, einen mechanischen Schutz des zu schützenden Bereichs zu sichern, wobei das das Klebeteil bildende Polymer-Gel ein größeres Haftvermögen aufweist, als das das Schutzteil bildende Gel, um das Plättchen auf dem zu schützenden Bereich zu halten.

2. Vorrichtung nach Anspruch 1, bei der das Schutzteil (2) des Plättchens (1, 1') eine Dicke zwischen 1 und 4 mm aufweist.

3. Vorrichtung nach Anspruch 1 oder 2, bei der das Klebeteil (3a, 3b) eine Dicke zwischen 0,2 und 0,5 mm aufweist.

4. Vorrichtung nach einem der Ansprüche 1 bis 3, bei der das Schutzteil (2) durch eine wenigstens teilweise Polymerisierung eines Gemischs aus Folgendem hergestellt wird:
etwa 15% Polydimethylsiloxan mit Dimethyl-Vinylendgruppen, das eine Viskosität größer als 20000 mPa.s aufweist,
etwa 25% Polydimethylsiloxan mit Dimethyl-Vinylendgruppen, das eine Viskosität zwischen 200 und 20000 mPa.s aufweist,
etwa 45% Polydimethylsiloxan mit Trimethyl-Endgruppen, das eine Viskosität kleiner als 100 mPa.s aufweist,
etwa 12% Trimethylsiloxy-behandeltes pyrogenes Siliziumdioxid, und
etwa 3% co-Polydimethylsiloxan-Polymethyl-Hydrogeno-Siloxan mit Dimethyl-Hydrogeno-Endgruppen.

5. Vorrichtung nach einem der Ansprüche 1 bis 3, bei der das Schutzteil (2) durch eine wenigstens teilweise Polymerisierung eines Gemischs aus Folgendem hergestellt wird:
etwa 15% Polydimethylsiloxan mit Dimethyl-Vinylendgruppen, das eine Viskosität größer als 20000 mPa.s aufweist,
etwa 25% Polydimethylsiloxan mit Dimethyl-Vinylendgruppen, das eine Viskosität zwischen 200 und 20000 mPa.s aufweist,
etwa 25% Polydimethylsiloxan mit Trimethyl-Endgruppen, das eine Viskosität kleiner als 100 mPa.s aufweist,
etwa 20% Polydimethylsiloxan mit Trimethyl-Endgruppen, das eine Viskosität größer als 20000 mPa.s aufweist,
etwa 12% Trimethylsiloxy-behandeltes pyrogenes Siliziumdioxid, und
etwa 3% co-Polydimethylsiloxan-Polymethyl-Hydrogeno-Siloxan mit Dimethyl-Hydrogeno-Endgruppen.

6. Vorrichtung nach einem der Ansprüche 1 bis 5, bei der die Außenschicht (4) aus Gewebe ist und auf einer Seite des Schutzteils (2) und des Klebeteils (3a, 3b) geklebt ist.

7. Vorrichtung nach Anspruch 6, bei der das die Außenschicht (4) bildende Gewebe elastisch ist.

8. Vorrichtung nach einem der Ansprüche 1 bis 5, bei der die Außenschicht (5) aus einem nicht klebenden Silicon-Gel mit einer Dicke zwischen 0,2 und 0,4 mm oder aus einem Siliconklebstoff mit einer Dicke zwischen 0,05 und 0,4 mm ist.

9. Vorrichtung nach einem der Ansprüche 1 bis 7, bei der jedes Klebeteil (3a, 3b) eine Breite zwischen 5 und 60 mm und ein Haftvermögen in der Größenordnung von 130 bis 140 g/cm2 aufweist.

10. Verfahren zur Herstellung einer Vorrichtung zum Schutz eines Bereichs der Haut des menschlichen Körpers, umfassend Schritte der Bildung eines Plättchens (1, 1') aus einem Polymer-Gel, und der Befestigung einer Außenschicht (4, 5) auf einer Seite (22, 32) des Plättchens, wobei das Plättchen ein Schutzteil (2) aus einem Polymer-Gel und wenigstens ein klebendes Seitenteil (3a, 3b) aus einem Polymer-Gel umfasst, die bestimmt sind, in Kontakt mit der Haut zu kommen, wobei die das Schutzteil und das Klebeteil bildenden Polymer-Gele wenigstens teilweise in einem Schnittstellenbereich zwischen dem Schutzteil und dem Klebeteil vermischt sind, wobei das Schutzteil so konfiguriert ist, einen mechanischen Schutz des zu schützenden Bereichs zu sichern, wobei das das Klebeteil bildende Polymer-Gel ein größeres Haftvermögen aufweist, als das das Schutzteil bildende Gel, um das Plättchen auf dem zu schützenden Bereich zu halten.

11. Verfahren nach Anspruch 10, umfassend Schritte der Bildung des Klebeteils (3a, 3b) aus einem ersten flüssigen, auf einen Träger aufgetragenen Gemisch (10), und der Bildung des Schutzteils (2) aus einem zweiten flüssigen, auf den Träger aufgetragenen Gemisch, wobei das erste Gemisch und das zweite Gemisch sich in einem Schnittstellenbereich (7a, 7b) vor der kompletten Polymerisierung der zwei Gemische teilweise mischen.

12. Verfahren nach Anspruch 11, bei dem das zweite Gemisch Folgendes umfasst:
etwa 15% Polydimethylsiloxan mit Dimethyl-Vinylendgruppen, das eine Viskosität größer als 20000 mPa.s aufweist,
etwa 25% Polydimethylsiloxan mit Dimethyl-Vinylendgruppen, das eine Viskosität zwischen 200 und 20000 mPa.s aufweist,
etwa 45% Polydimethylsiloxan mit Trimethyl-Endgruppen, das eine Viskosität kleiner als 100 mPa.s aufweist,
etwa 12% Trimethylsiloxy-behandeltes pyrogenes Siliziumdioxid, und
etwa 3% co-Polydimethylsiloxan-Polymethyl-Hydrogeno-Siloxan mit Dimethyl-Hydrogeno-Endgruppen.

13. Verfahren nach Anspruch 11, bei dem das zweite Gemisch Folgendes umfasst:
etwa 15% Polydimethylsiloxan mit Dimethyl-Vinylendgruppen, das eine Viskosität größer als 20000 mPa.s aufweist,
etwa 25% Polydimethylsiloxan mit Dimethyl-Vinylendgruppen, das eine Viskosität zwischen 200 und 20000 mPa.s aufweist,
etwa 25% Polydimethylsiloxan mit Trimethyl-Endgruppen, das eine Viskosität kleiner als 100 mPa.s aufweist,
etwa 20% Polydimethylsiloxan mit Trimethyl-Endgruppen, das eine Viskosität größer als 20000 mPa.s aufweist,
etwa 12% Trimethylsiloxy-behandeltes pyrogenes Siliziumdioxid, und
etwa 3% co-Polydimethylsiloxan-Polymethyl-Hydrogeno-Siloxan mit Dimethyl-Hydrogeno-Endgruppen.

14. Verfahren nach Anspruch 13, bei dem die beiden flüssigen Gemische auf einen laufenden Träger (10) so aufgetragen werden, dass Streifen gebildet werden, wobei das erste Gemisch auf den Träger so aufgetragen wird, dass zwei klebende Streifen (3a, 3b) gebildet werden, zwischen denen das zweite Gemisch zur Bildung des Schutzteils (2) in Form von Streifen aufgetragen wird.

15. Verfahren nach Anspruch 14, bei dem die Dicke und die Breite jedes Schutz- (2) und Klebeteils (3a, 3b) durch eine Leiste (16, 19) und Seitenführungen (15a, 15b) angepasst werden.

16. Verfahren nach einem der Ansprüche 10 bis 15, umfassend einen Schritt der Befestigung einer nicht klebenden Außenschicht (4, 5) auf einer Seite des Plättchens (1, 1'), die gegenüber der mit der Haut in Kontakt zu kommenden Seite (21, 31) liegt.

17. Verfahren nach Anspruch 16, umfassend einen Schritt des Klebens der aus Gewebe bestehenden Außenschicht (4) auf das Plättchen (1).

18. Verfahren nach Anspruch 16, bei dem die Außenschicht (5) aus einem nicht klebenden Polymer-Gel mit einer Dicke zwischen 0,2 und 0,4 mm oder aus einem Siliconklebstoff mit einer Dicke zwischen 0,05 und 0,4 mm gebildet wird.

19. Verfahren nach einem der Ansprüche 10 bis 18, umfassend einen Schritt der Bedeckung der mit der Haut in Kontakt zu kommenden Seite (21, 31) des Plättchens (1, 1') mit einem Schutzfilm (6).
